(19) Europäisches Patentamt
European Patent Office
Office européen des brevets

(11) EP 1 854 452 A1

(12) # DEMANDE DE BREVET EUROPEEN

(43) Date de publication:
**14.11.2007 Bulletin 2007/46**

(21) Numéro de dépôt: **07009359.6**

(22) Date de dépôt: **10.05.2007**

(51) Int Cl.:
***A61K 8/97*** *(2006.01)* ***A61K 36/28*** *(2006.01)*
***A61K 36/41*** *(2006.01)* ***A61Q 19/00*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **10.05.2006 FR 0604119**

(71) Demandeur: **Laboratoires Dermatologiques d'Uriage**
**92404 Courbevoie Cedex (FR)**

(72) Inventeur: **Lefeuvre, Luc**
**78580 Bazemont (FR)**

(74) Mandataire: **Burtin, Jean-François**
**Cabinet GEFIB,**
**55 rue Aristide Briand**
**92309 Levallois-Perret Cedex (FR)**

(54) **Compositions cosmétiques ou dermatologiques à base d'extraits de Vernonia sublutea**

(57) La présente invention a spécifiquement pour objet l'utilisation à des fins cosmétiques ou dermatologiques d'un extrait de *Vernonia sublutea.*

La présente invention a également pour objet la réalisation de compositions cosmétiques ou dermatologiques contenant, à titre de principe actif, au moins un extrait de *Vernonia sublutea*, pour le traitement préventif ou curatif de l'hypersensibilité cutanée, du vieillissement cutané et en réparation cutanée.

EP 1 854 452 A1

**Description**

La présente invention se rapporte au domaine des nécessités de la vie et plus particulièrement au domaine de la cosmétologie et de la dermatologie.

La présente invention se rapporte plus particulièrement à l'utilisation d'extraits de plantes à des fins cosmétiques ou dermatologiques.

La présente invention a spécifiquement pour objet l'utilisation à des fins cosmétiques ou dermatologiques d'un extrait de *Vernonia sublutea.*

La présente invention a également pour objet la réalisation de compositions cosmétiques ou dermatologiques contenant, à titre de principe actif, au moins un extrait de *Vernonia sublutea.*

La *Vernonia sublutea* appartient à la famille des Astéracées. Le genre des Vemonia renferme plus de mille espèces à travers les régions tropicales et chaudes, jusqu'en Amérique du Nord. Plusieurs principes actifs contenus par diverses espèces de Vemonia ont déjà été décrits, notamment des sesquiterpène lactones ayant une activité antitumorale ou antihépatotoxique (Babalola O.O. et. al., Afr. J. Med. Med. Sci. 2001, 91-93). Des extraits de Vernonia comme *Vernonia cinerea* ou *Vernonia scorpioides* ont d'autre part montré une activité anti-inflammatoire ou cicatrisante (Mazumder U.K. et. al., Phytomedicine 2003, 185-188 ; Leite S.N. et. al., Fitoterapia 2002, 73, 496-500).

*Vernonia sublutea* est utilisée en médecine traditionnelle à Madagascar, notamment pour ses propriétés tonifiantes de la peau.

Bien que certaines espèces apparentées soient connues, aucune littérature spécifique ne décrit les propriétés cosmétiques ou dermatologiques de *Vernonia sublutea.* Un composé original, le guaïanolide (lactone sesquiterpénique), isolé des feuilles de *Vernonia sublutea* a été décrit, sans mentionner d'activité spécifique (Mompon B., Toubiana R., Tetrahedron 1976, 33, 2199-2203).

Or, la Demanderesse a constaté que des extraits de *Vernonia sublutea* présentent des propriétés intéressantes, notamment en terme d'activité anti-inflammatoire et cicatrisante. D'une manière générale, les extraits de *Vernonia sublutea* ont une toxicité très faible ; ils sont aptes à une utilisation cosmétique ou dermatologique.

Un objet de la présente invention est l'utilisation d'un extrait de *Vernonia sublutea,* pur ou sous la forme d'une composition, à des fins cosmétiques ou dermatologiques.

Les utilisations cosmétiques et dermatologiques considérées pour ces extraits de plantes concernent notamment le traitement de l'hypersensibilité cutanée, du vieillissement cutané et la réparation cutanée.

Les utilisations cosmétiques comprennent, d'une manière générale et non exhaustive, le traitement préventif et/ou curatif des peaux sensibles et intolérantes, des peaux hyper-réactives, des peaux sujettes aux rougeurs et des peaux grasses à imperfections. Elles comprennent également le traitement préventif et/ou curatif des peaux marquées par les signes du vieillissement, tels que l'amincissement de la peau, les rides et ridules, le flétrissement de la peau ou le manque d'élasticité.

Les utilisations dermatologiques comprennent, de manière non exhaustive, l'usage dans les cas de dermatite atopique (eczema), de psoriasis, de prurit, de rosacée, de dermite séborrhéique, d'acné et pour la cicatrisation des plaies.

Un autre objet de la présente invention est la réalisation de compositions cosmétiques ou dermatologiques destinées à une application topique, contenant, à titre de principe actif, au moins un extrait de *Vernonia sublutea,* en association ou en mélange avec au moins un excipient et/ou véhicule approprié.

Un extrait de *Vernonia sublutea* utilisé selon l'invention peut être préparé à partir de matériel végétal issu de la plante entière ou de partie de plante comme les feuilles, les tiges, les fleurs, les pétales, les fruits ou les racines.

Selon une forme préférentielle de l'invention, un extrait de *Vernonia sublutea* est préparé à partir de matériel végétal issu des feuilles et des tiges de la plante.

Toute méthode d'extraction connue de l'homme du métier peut être utilisée pour préparer un extrait de *Vernonia sublutea* utilisé selon l'invention.

On peut notamment utiliser les méthodes d'extraction par des solvants comme l'eau, les alcools ou les solvants organiques. On peut également utiliser des mélanges de ces différents solvants, comme par exemple des mélanges hydroalcooliques. Les extractions peuvent notamment avoir lieu à température ambiante ou à une température plus élevée, selon le point d'ébullition du solvant utilisé.

Préférentiellement, les extractions ont lieu à une température comprise entre 20 et 70 °C.

Parmi les alcools pouvant être utilisés comme solvant d'extraction, on peut notamment citer l'éthanol.

Parmi les solvants organiques pouvant être utilisés comme solvant d'extraction, on peut notamment citer l'acétate d'éthyle, le dichlorométhane et les alcanes comme l'hexane, le cyclohexane, l'heptane ou l'octane.

On peut également réaliser une série d'extractions successives par différents solvants, chaque phase d'extraction étant concentrée et le concentrât étant soumis à une nouvelle extraction par un solvant différent. Cette méthode permet de réaliser plusieurs extraits au contenu spécifique, pouvant présenter des propriétés originales.

Par exemple, on peut effectuer une première extraction du matériel végétal à l'aide d'un solvant alcoolique ou hydroalcoolique comme l'éthanol ou un mélange éthanol/eau, concentrer la phase d'extraction obtenue et effectuer une

extraction du concentrât à l'aide d'un solvant organique, comme un alcane ou l'acétate d'éthyle. Cette opération peut être répétée plusieurs fois à l'aide de différents solvants.

Selon une forme de l'invention, un extrait de *Vernonia sublutea* constitue le principe actif des compositions selon l'invention.

Selon une autre forme de l'invention, le principe actif des compositions selon l'invention est constitué de l'association d'un extrait de *Vernonia sublutea* avec un ou plusieurs autres composés naturels ou synthétiques, purs ou sous forme d'extraits végétaux, d'action complémentaire, similaire ou synergique.

Selon une forme préférentielle de l'invention, le principe actif des compositions selon l'invention est constitué de l'association d'un extrait de *Vernonia sublutea* avec un extrait de *Kalanchoe linearifolia.* Cette plante appartient à la famille des Crassulacées. Le genre Kalanchoe comprend approximativement 120 espèces, localisées majoritairement en Afrique, plus particulièrement à Madagascar. Des extraits de Kalanchoe comme *Kalanchoe brasiliensis* ont déjà montré une activité anti-inflammatoire (Mourão R.H. et. al., Phytotherapy Research 1999, 352-354).

Selon une autre forme préférentielle de l'invention, le principe actif des compositions selon l'invention est constitué de l'association d'un extrait de *Vernonia sublutea* avec un extrait de *Sida acuta.* Cette plante tropicale appartient à la famille des Malvacées. Des extraits de cette plante présentent également des propriétés anti-inflammatoires et cicatrisantes.

Selon une forme préférentielle de l'invention, le principe actif des compositions selon l'invention est constitué de l'association d'un extrait de *Vernonia sublutea* avec un extrait de *Kalanchoe linearifolia* et un extrait de *Sida acuta.*

Un extrait de *Kalanchoe linearifolia* ou de *Sida acuta* utilisé selon l'invention peut être préparé à partir de matériel végétal issu de la plante entière ou de partie de plante comme les feuilles, les tiges, les fleurs, les pétales, les fruits ou les racines.

Selon une forme préférentielle de l'invention, un extrait de *Kalanchoe linearifolia* ou de *Sida acuta* est préparé à partir de matériel végétal issu des feuilles et des tiges de la plante.

Toute méthode d'extraction connue de l'homme du métier peut être utilisée pour préparer un extrait de *Kalanchoe linearifolia* ou de *Sida acuta* utilisé selon l'invention, notamment les méthodes décrites ici pour la préparation d'extraits de *Vernonia sublutea.*

Une composition contenant au moins un extrait de *Vernonia sublutea* est utilisée selon l'invention en application topique sur la peau et/ou les ongles et/ou les cheveux et/ou les muqueuses externes.

La quantité d'extrait utilisable selon l'invention peut varier dans une large mesure en fonction de l'effet recherché.

Pour donner un ordre de grandeur, on peut utiliser l'extrait à l'état pur en une quantité représentant de 0,0001 à 20% du poids total de la composition, et préférentiellement en une quantité représentant de 0,01 à 10% du poids total de la composition.

Les compositions selon l'invention comprennent un support cosmétiquement ou dermatologiquement acceptable, en association ou en mélange avec le principe actif. Elles peuvent renfermer les adjuvants couramment utilisés dans les domaines cosmétiques et dermatologiques, comme par exemple des agents émollients ou adoucissants, des gélifiants, des antioxydants, des solvants, des filtres, des conservateurs, des pigments ou des parfums. Elles peuvent se présenter sous une forme compatible avec une application topique, comme les solutions aqueuses, hydroalcooliques ou huileuses, les dispersions, les suspensions, les émulsions huile dans eau ou eau dans huile, les gels aqueux ou huileux, les crèmes, les laits, les pommades, les lotions, les pâtes, les poudres, les mousses, les sticks ou les aérosols.

Les propriétés pharmacologiques, notamment anti-inflammatoires et cicatrisantes des compositions selon l'invention, conduisent à des utilisations cosmétiques telles que le traitement préventif et/ou curatif des troubles de la peau, comme par exemple les peaux sensibles et intolérantes, les peaux hyper-réactives, les peaux sujettes aux rougeurs et/ou les peaux grasses à imperfections, ainsi que le traitement préventif et/ou curatif des peaux marquées par les signes du vieillissement, comme la peau amincie, les rides et ridules, la peau flétrie ou manquant d'élasticité.

Les propriétés notamment anti-inflammatoires et cicatrisantes des compositions selon l'invention conduisent à des utilisations dermatologiques telles que l'usage dans les cas de dermatite atopique (eczema), de psoriasis, de prurit, de rosacée, de dermite séborrhéique, d'acné et pour la cicatrisation des plaies.

De manière préférée, les compositions selon l'invention sont destinées principalement à un usage cicatrisant. Elles contiennent au moins un extrait de *Vernonia sublutea,* éventuellement associé à un extrait de *Sida acuta* et/ou de *Kalanchoe linearifolia.*

Les exemples suivants illustrent l'invention sans toutefois la limiter :

**Exemple 1 : Réalisation d'un extrait de plante utilisé selon l'invention**

Ces méthodes peuvent être appliquées aussi bien à la réalisation d'un extrait de *Vernonia sublutea* qu'à la réalisation d'un extrait de *Kalanchoe linearifolia* ou de *Sida acuta.* La matière végétale utilisée est issue des feuilles et des tiges de la plante.

**Exemple 1.1 : Réalisation de l'extrait brut (fraction f1) de *Vernonia sublutea***

1100 g de matière végétale séchée sont dispersés dans 5 L d'éthanol à 70°. Le mélange est agité durant 1 heure à 50 °C, puis filtré sur entonnoir, le résidu solide étant rincé par 2 L d'éthanol. La phase alcoolique est concentrée sous pression réduite. Le résidu est repris dans 1,5 litre d'eau et lyophilisé. 10 g d'un solide brun sont obtenus. Dans les exemples suivants, cet extrait sera dénommé « fraction f1 ».

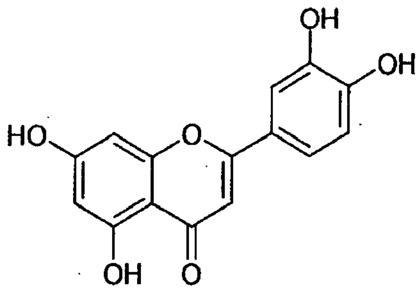

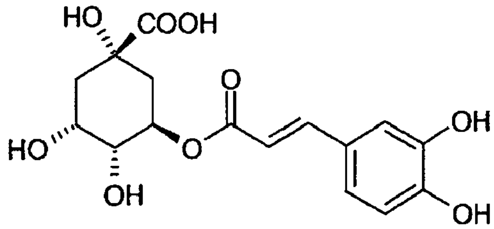

Lutéoline [491-70-3] Acide chlorogénique [327-97-9]

Les molécules suivantes ont été identifiées dans l'extrait f1 de *Vernonia sublutea* :

(Flavonoïde) (Acide phénolique)

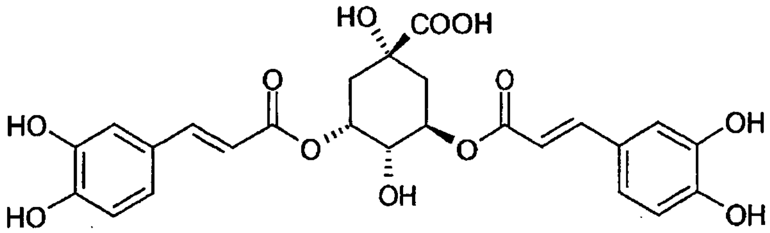

Acide 3,5-dicafféolyquinique

(Acide phénolique)

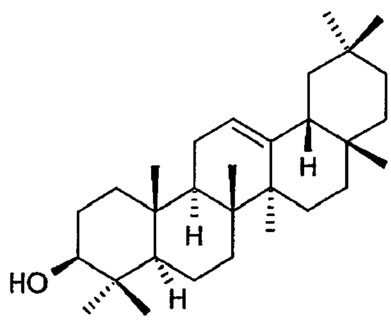

β-Amyrine[559-70-6]

(Triterpène)

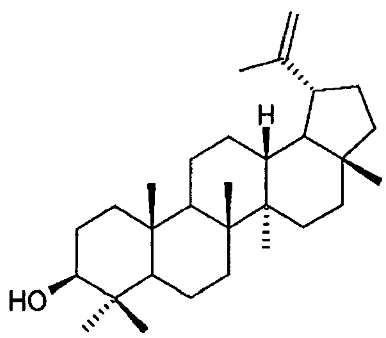

Lupéol [545-47-1]

(Triterpène)

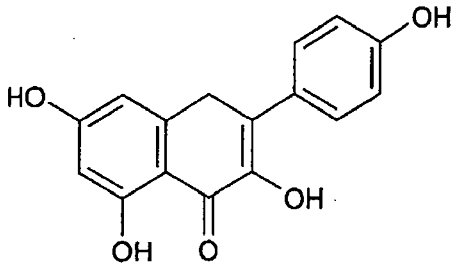

Kaempférol [520-18-3]
(Flavonoïde)

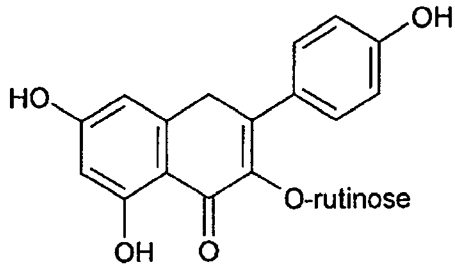

Rutine [153-18-4]
(Flavonoïde)

La fraction f1 de *Kalanchoe linearifolia* est obtenue de manière similaire.

**Exemple 1.2 : Réalisation de l'extrait à l'aide de solvants peu polaires (fraction f2) de *Vernonia sublutea***

La fraction f1 précédemment obtenue est mise en solution dans 500 mL d'éthanol. La solution est extraite par 3 x 200 mL d'hexane. Les phases hexaniques sont rassemblées puis concentrées. 2,9 g d'un produit visqueux sont obtenus. Dans les exemples suivants, cet extrait sera dénommé « fraction f2 ».

La fraction f2 de *Kalanchoe linearifolia* est obtenue de manière similaire.

**Exemple 1.3 : Réalisation de l'extrait à l'aide d'un solvant de polarité intermédiaire (fraction f3) de *Vernonia sublutea***

La phase éthanolique de l'étape précédente est concentrée sous pression réduite et reprise dans 500 mL d'eau. Cette solution aqueuse est extraite par 3 * 200 mL d'acétate d'éthyle. Les phases organiques sont rassemblées puis concentrées. 1,1 g d'un produit visqueux sont obtenus. Dans les exemples suivants, cet extrait sera dénommé « fraction f3 ».

La fraction f3 de *Kalanchoe linearifolia* est obtenue de manière similaire.

**Exemple 1.4 : Réalisation d'un extrait hydrosoluble (fraction f4) de *Vernonia sublutea***

La phase aqueuse de l'étape précédente est lyophilisée. 13,8 g d'un solide brun sont obtenus. Dans les exemples suivants, cet extrait sera dénommé « fraction f4 ».

La fraction f4 de *Kalanchoe linearifolia* est obtenue de manière similaire.

**Exemple 1.5 : Réalisation d'un extrait (fraction f5)de *Vernonia sublutea***

100 g de matière végétale séchée sont dispersés dans 2,5 L d'heptane. La suspension est soumise à une extraction au soxhlet à reflux du solvant pendant 4 heures. Après filtration sur entonnoir, la matière végétale est récupérée et ajoutée à 1 L d'éthanol à 70°. Le mélange est agité durant 7 heures au reflux du solvant, puis filtré sur entonnoir. Le résidu solide est ajouté à 1 L d'éthanol à 70° et le mélange est laissé au repos durant 3 jours. Après filtration, les phases alcooliques sont rassemblées et concentrées sous pression réduite. Le résidu est repris dans 1,5 litre d'eau et lyophilisé. 6 g d'un solide brun sont obtenus. La composition de cet extrait est très proche de celle de la fraction f3. La fraction f5 de *Kalanchoe linearifolia* est obtenue de manière similaire.

**Exemple 2 : Evaluation de l'activité anti-inflammatoire d'extraits de *Vernonia sublutea* et d'extraits de *Kalanchoe linearifolia***

1- OBJECTIFS

Il s'agit de tester les fractions de *Vernonia sublutea* (VSU) et de *Kalanchoe linearifolia* (KLN) dans un test discriminatoire anti-inflammatoire. Les fractions VSU f1, f2, f3, f4 et les fractions KLN f1, f2, f3, f4 ont été étudiées.

2-CONDITIONS EXPERIMENTALES

L'inflammation est provoquée par injection intra-péritonéale d'une solution d'acide acétique 0,6% qui provoque une inflammation du réseau mésentérique chez la souris Swiss. Cet état inflammatoire se traduit par une augmentation de la perméabilité capillaire de la paroi endothéliale avec pour conséquence un chimiotactisme accru. Le témoin de cette augmentation de la perméabilité, est le Bleu Evans, colorant injecté au préalable par voie intraveineuse dans la veine caudale des souris. Une substance à caractère anti-inflammatoire réduit cette perméabilité et par conséquent l'invasivité par le colorant de la cavité abdominale (mesure par D.O.)

3-METHODOLOGIE

3.1 -Plan expérimental

1 dose unique a été testée dans cette étude: 1g/kg (n = 6)
la concentration en Bleu Evans (BE) sera déterminée par une méthode colorimétrique. Le produit de référence utilisé pour évaluer les extraits est la phénylbutazone (PNB) à la dose de 100 mg/kg.

3.2-Critère d'*év*aluation

La réduction de l'inflammation se traduit par une diminution du passage du colorant (Bleu d'Evans) dans la cavité abdominale donc de sa concentration dans l'exsudat.

3.3-Pertinence de la méthode

Ce test est très utilisé pour évaluer un nouvel anti-inflammatoire en première intention. Il a été décrit par Whittle pour la première fois en 1964 et est encore largement utilisé en screening d'évaluation.

3.4-Déroulement de l'essai

*3.4.1 Race de souris utilisée*

Des souris des deux sexes, de race Swiss pesant entre 27 et 30 g ont été utilisées.
Dix huit heures avant l'expérience, les animaux ont été mis à jeun, tout en ayant un accès libre à l'eau.
Les animaux ont été répartis par lot de 6 souris, un lot ayant reçu uniquement de l'eau distillée, a servi de lot témoin.

*3.4.2 Voie d'administration des extraits*

Ces extraits ont été administrés par voie orale, par gavage. Ils ont été dilués dans de l'eau distillée à la dose de 1 g/kg. 4 lots de traités sont ainsi constitués.
La phénylbutazone (PNB) a été utilisée à la dose de 100 mg/kg comme produit de référence.

3.5 -Protocole expérimental

*3.5.1 Perméabilité capillaire*

45 minutes après l'administration des extraits, une solution de colorant (du bleu d'Evans : BE) à la concentration de 0.5% préparée dans une solution physiologique de NaCl (0.9%) a été injectée par voie intraveineuse dans la veine caudale des souris à raison de 10 ml/kg de poids corporel.
15 minutes après l'injection du BE, une solution d'acide acétique (0.6%) a été injectée par voie intra-péritonéale à raison de 10 ml/kg.
Après 30 minutes, les animaux ont été sacrifiés et leur cavité abdominale a été rincée avec 5 ml d'eau distillée contenant 500 UI d'héparine. L'eau de rinçage a été quantitativement collectée et centrifugée à la vitesse de 2000 t/min
La concentration en BE dans le surnageant a été déterminée à l'aide d'un spectrophotomètre à la longueur d'onde de 620 nm (Whittle, 1964).
L'activité inhibitrice des extraits de plantes ainsi que celle de la substance de référence a été exprimée en pourcentage de la perméabilité capillaire chez les animaux traités par rapport au témoin.

*3.5.1 Calcul du pourcentage d'inhibition*

$$\text{Pourcentage d'inhibition} = 1 - (DO_2/DO_1 \times 100)$$

Où:
$DO_1$ = la moyenne de la densité optique du BE chez les animaux témoins
$DO_2$= la moyenne de la densité optique du BE chez les animaux traités.

Les activités des différents extraits ont été comparées à celle du PNB.

4- RESULTATS ET DISCUSSION : VSU

Le tableau 1 ci-dessous montre les variations individuelles de l'inhibition de la perméabilité capillaire provoquée par l'acide acétique injecté par voie i.p. chez la souris, après administration per os de différentes fractions. Chaque valeur est la moyenne de 6 observations.

*Tableau 1*

| EXTRAIT n=6 | PNB (0,1 g) | VSU f1 | VSU f2 | VSU f3 | VSU f4 | Excipient |
|---|---|---|---|---|---|---|
| % Inhibition Moyenne ± s.e.m. | 58,75 ±11,6 | 21,7 ±3 | 17,7 ±2,1 | 25,2 ±3,6 | 2,5 ±5,6 | -1,9 ±0,98 |
| *'t'//témoins* | *P<0,05* | *P<0,05* | *NS* | *P<0,05* | *NS* | *NS* |

Les fractions VSU f1 et f3 testées dans cet essai témoignent d'une activité anti-inflammatoire dans le cadre de ce test de screening, à la dose élevée de 1g/kg (extraits bruts non connus quant à leur teneur en actifs).
Dans ce test, le PNB à la dose de 0.1 g/kg provoque une inhibition de l'inflammation à l'acide acétique à la valeur de 58,75% par rapport au groupe d'animaux témoins non traités.
L'effet anti-inflammatoire des fractions f1 et f3 est modéré mais réel. Les fractions f2 et f4 doivent être considérées comme inactives.

Conclusion:

L'activité anti-inflammatoire de VSU est confirmée, surtout en ce qui concerne la fraction f3. Cette propriété est toutefois modérée.

5- RESULTATS ET DISCUSSION : KLN

Le tableau 2 ci-dessous montre les variations individuelles de l'inhibition de la perméabilité capillaire provoquée par l'acide acétique injecté par voie i.p. chez la souris, après administration per os de différentes fractions. Chaque valeur est la moyenne de 6 observations.

*Tableau 2*

| EXTRAIT n = 6 | PNB (0,1 g) | KLN f1 | KLN f2 | KLN f3 | KLN f4 | Excipient témoins |
|---|---|---|---|---|---|---|
| % Inhibition Moyenne ± s.e.m. | 71,9 ±9,3 | 53,9 ±2,9 | 17,7 ±4,7 | 61,3 ±6,3 | 33 ±6,6 | -3,1 ±1,22 |
| *'t'//témoins* | P<0,01 | *P<0,05* | *NS* | *P<0,01* | *P<0,05* | *NS* |

Les fractions KLN f1 et f3 testées dans cet essai témoignent d'une activité anti-inflammatoire dans le cadre de

ce test de screening, à la dose élevée de 1g/kg (extraits bruts non connus quant à leur teneur en actifs).
Dans ce test, le PNB à la dose de 0.1 g/kg provoque une inhibition de l'inflammation à l'acide acétique à la valeur de 71,9% par rapport au groupe d'animaux témoins non traités (eux-mêmes ayant eu une augmentation de leur perméabilité capillaire de 120% par rapport aux animaux normaux après injection d'acide acétique).

L'extrait brut f1 et la fraction f3 sont anti-inflammatoires de façon significative. La modération de la perméabilité cellulaire atteste de l'efficacité de f1 du fait des molécules sélectionnées dans f3.
Les fractions f2 et f4 doivent être considérées comme peu actives dans ce test, bien que f4 soit significativement différent du lot des animaux témoins au stade inflammatoire.

Conclusion :

L'activité anti-inflammatoire de KLN est confirmée, surtout en ce qui concerne la fraction f3.

**Exemple 3 : Evaluation de l'activité cicatrisante d'extraits de *Vernonia sublutea* et de *Kalanchoe linearifolia***

1- OBJECTIFS

Evaluér comparativement *in vivo* chez la souris la potentialité accélératrice de la néoformation de tissus dermiques et épidermiques au moyen des fractions de *Vernonia sublutea* (VSU) et de *Kalanchoe linearifolia* (KLN) f1, f2, f3, f4, en comparaison avec :

- un groupe recevant un produit de référence (TECA 1% - principe actif du Madécassol® ROCHE-NICHOLAS),
- un groupe traité uniquement par l'excipient des extraits,
- un groupe neutre ne recevant aucun traitement.

2-CONDITIONS EXPERIMENTALES

Les plaies ont été obtenues par excision de la peau après une légère anesthésie à l'éther. La surface initiale des plaies est de 12,5 $mm^2$, délimitées avec un tampon d'encre de diamètre égal à 4mm.
Les extraits ont été préparés sous forme d'onguent, avec pour excipient, un mélange de lanoline-vaseline-eau (40 / 50 / 10), à un concentration de 10% pour les extraits f1. L'extrait f1 est expérimenté à 10%, les fractions f2 à f4, à 5% et le TECA à 1 %.

3-METHODOLOGIE

3.1 - Plan expérimental

- Période de préparation des animaux en expérience (10 jours)
- Préparation des onguents (2 jours)
- Durée prévue de l'expérimentation (4 semaines)
- Exploitation des résultats (1 semaine).

3.2 - Critères d'évaluation

- Durée de la cicatrisation totale
- Evolution de la surface des plaies

3.3 - Pertinence de la méthode

L'évaluation de l'effet d'un cicatrisant dans le processus réparatoire naturel, peut être faite avec une méthode telle que l'excision linéaire. Toutefois, le choix s'est porté sur la technique de la plaie circulaire qui présente l'avantage d'une meilleure précision de mesure, du fait de la continuité du processus de reconstitution jusqu'au stade de fermeture totale de la plaie

3.4 - Déroulement de l'.essai

*3.4.1-Manipulation des animaux*

Par série de manipulations, les extraits ont été évalués systématiquement par rapport au produit de référence, le TECA, et les deux groupes témoins évoqués en 1.
Après rasage, les animaux sélectionnés (Souris Swiss : 4 mâles et femelles n = 8 d'un poids de 23 grammes +/-3) sont légèrement anesthésiés à l'éther, puis les excisions de la peau sont faites avec une paire de ciseaux chirurgicaux.
Les plaies sont pratiquées au niveau de l'omoplate, région non accessible au grattage.
Chaque animal est placé dans une cage individuelle, où il reçoit de la nourriture granulée et de l'eau à volonté, durant tout le traitement jusqu'à la cicatrisation totale.
Les applications ont débuté 24 heures après l'excision.

*3.4.2-Evaluation de la cicatrisation*

Avant chaque mesure, les croûtes sont humidifiées avec de l'eau distillée et enlevées pour la précision des mesures.
Les extraits (ou le produit de référence) sont appliqués sur les plaies quotidiennement (7/7 jours), par étalement en couche fine et continue de l'onguent au moyen d'une spatule.
Le relevé de la surface des plaies a été fait par planimétrie directe sur un papier millimétré semi-transparent.

3.5 - Analyse des résultats

Les résultats obtenus ont été analysés statistiquement par analyse de variance suivi d'un "t" test de Student. Le degré de signification a été fixé pour S à une valeur de P≤0,05% et HS à 0,01 %.
Le paramètre d'évaluation est le temps total qui correspond à 100% de cicatrisation, c'est à dire de la fermeture complète des plaies.
L'activité du VSU et celle du KLN ont été comparées statistiquement à celle du TECA, ainsi qu'aux témoins non traités.

4- RESULTATS ET DISCUSSION : VSU

Les résultats obtenus après application journalière des préparations d'extraits ainsi que de l'excipient sont résumés dans le Tableau 3 : durée moyenne de la cicatrisation à 100% des plaies provoquées par excision de la peau chez la souris (moyenne ± écart standard à la moyenne, n = 8).

*Tableau 3*

| Produit | TECA | VSU f1 | VSU f2 | VSU f3 | VSU f4 | Excipient | Neutre |
|---|---|---|---|---|---|---|---|
| Moyenne (jours) | 15 | 22 | 24 | 19 | 26 | 30 | 33 |
| s.e.m. | 0,65 | 0,87 | 1,23 | 0,96 | 1,30 | 1,27 | 1,05 |
| 't' Student // TECA | - | NS | P<0,05 | NS | P<0,01 | P<0,01 | P<0,01 |
| 't' Student // témoins P<0,05 | P<0,01 | P<0,05 | NS | P<0,05 | NS | - | - |

4.1 - Groupe traité avec l'excipient et groupe non traité

Le traitement des souris avec l'excipient seul montre une durée de cicatrisation totale de 25 jours, contre 28 jours pour le groupe de souris non traité, durée de cicatrisation maximum selon ce protocole. La comparaison de ces 2 groupes montre une différence non significative.

4.2 - Comparaison de l'activité des fractions avec le produit de référence

VSU (5%), l'extrait brut f1 a témoigné d'une activité cicatrisante moyenne mais significative (raccourcissement du temps de 26,6% contre 50% pour le TECA). La fraction la plus active est f3, qui permet une cicatrisation complète en 19 jours.

4.3 - Cinétiques de cicatrisation

La cicatrisation est progressive aussi bien en qui concerne le TECA, que la fraction f3 de VSU. C'est entre le 7ème et le 15ème jour que le TECA distance VSU dans la phase de comblement. Dans la phase tardive de reconstitution du l'épiderme, les cinétiques sont comparables.

Conclusion :

L'activité cicatrisante de VSU est confirmée, surtout en ce qui concerne la fraction f3.

5- RESULTATS ET DISCUSSION : KLN

Les résultats obtenus après application journalière des préparations d'extraits ainsi que de l'excipient sont résumés dans le Tableau 4 : durée moyenne de la cicatrisation à 100% des plaies provoquées par excision de la peau chez la souris (moyenne $\pm$ écart standard à la moyenne, n = 8).

*Tableau 4*

| Produit | TECA | KLN f1 | KLN f2 | KLN f3 | KLN f4 | Excipient | Neutre |
|---|---|---|---|---|---|---|---|
| Moyenne (jours) | 14 | 24 | 2 1 | 23 | 24 | 25 | 28 |
| s.e.m. | 0,75 | 0,78 | 0,93 | 0,75 | 1,02 | 1,27 | 1,05 |
| 't' Student // TECA | P-<0,05 | P<0,05 | P<0,05 | P<0,05 | P<0,05 | P<0,01 | P<0,01 |
| 't' Student // témoins P<0,05 | P<0,01 | NS | NS | NS | NS | NS | NS |

5.1 - Groupe traité avec l'excipient et groupe non traité

Le traitement des souris avec l'excipient seul montre une durée de cicatrisation totale de 25 jours, contre 28 jours pour le groupe de souris non traité, durée de cicatrisation maximum selon ce protocole. La comparaison de ces 2 groupes montre une différence non significative.

5.2 - Comparaison de l'activité des fractions avec le produit de référence

KLN (5%), l'extrait brut f1 n'a témoigné d'aucune activité cicatrisante significative. Aucune des fractions f2 à f4 n'est différentiellement active.

5.3 - Cinétiques de cicatrisation

La cicatrisation est progressive aussi bien en qui concerne le TECA, que la fraction f3 de KLN. C'est entre le 7ème et le 15ème jour que le TECA distance KLN dans la phase de comblement. Dans la phase tardive de reconstitution du l'épiderme, les cinétiques sont comparables.

Conclusion :

L'activité cicatrisante de KLN n'est pas significative.

**Exemple 4 : Composition cosmétiques et dermatologiques contenant des extraits de *Vernonia sublutea* et de *Kalanchoe linearifolia***

Réalisation d'un onguent :

| **Dénomination INCI** | **Concentration (%)** |
|---|---|
| Petrolatum | 25 - 75 |

(suite)

| **Dénomination INCI** | **Concentration (%)** |
|---|---|
| Paraffinum liquidum | 15 - 30 |
| Polydecene | 5 - 20 |
| Dipentaerythrityl pentahydroxystearate / isostearate | 1 - 3 |
| Glycerin | 1 - 10 |
| *Vernonia sublutea* | 0,1 - 5 |
| *Kalanchoe linearifolia* | 0,1 - 5 |
| TOTAL | Qsp 100 |

Réalisation d'une émulsion :

| **Dénomination INCI** | **Concentration (%)** |
|---|---|
| Caprylic/capric triglyceride | 5 - 15 |
| Stearalkonium hectorite / propylene carbonate | 1 - 10 |
| Myristyl myristate | 1-5 |
| Hexyl decyl stearate | 5 - 15 |
| Isostearyl diglyceryl succinate | 1-10 |
| *Vernonia sublutea* | 0,1 - 5 |
| *Kalanchoe linearifolia* | 0,1 - 5 |
| Aqua | Qsp 100 |

Les extraits de plantes utilisés ici sont les fractions f5.

## Revendications

1. Compositions cosmétiques ou dermatologiques destinées à l'application topique, **caractérisées en ce qu'**elles renferment, à titre de principe actif, au moins un extrait de *Vernonia sublutea,* en association ou en mélange avec un excipient ou véhicule approprié.

2. Compositions cosmétiques ou dermatologiques selon la revendication 1, **caractérisées en ce qu'**elles renferment en outre un extrait de *Kalanchoe linearifolia*

3. Compositions cosmétiques ou dermatologiques selon la revendication 1 ou la revendication 2, **caractérisées en ce qu'**elles renferment en outre un extrait de *Sida acuta.*

4. Compositions cosmétiques ou dermatologiques selon l'une des revendications précédentes, **caractérisées en ce que** l'extrait ou les extraits sont obtenus selon un procédé impliquant une étape d'extraction de la plante ou d'une partie de la plante séchée, à l'aide d'un solvant alcoolique ou hydroalcoolique.

5. Compositions cosmétiques ou dermatologiques selon l'une des revendications précédentes, **caractérisées en ce que** la quantité de l'extrait ou de chacun des différents extraits est comprise entre 0,0001 et 20% du poids total de la composition.

6. Compositions cosmétiques ou dermatologiques selon l'une des revendications précédentes, **caractérisées en ce que** la quantité de l'extrait ou de chacun des différents extraits est comprise entre 0,01 et 10% du poids total de la

composition.

7. Compositions cosmétiques ou dermatologiques selon l'une des revendications précédentes, **caractérisées en ce qu'**elles renferment en outre un ou plusieurs principes actifs complémentaires.

Office européen des brevets

# RAPPORT DE RECHERCHE EUROPEENNE

Numéro de la demande
EP 07 00 9359

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | JOHRI R.K. AND SINGH C.: "Medicinal uses of Vernonia species" JOURNAL OF MEDICINAL AND AROMATIC PLANT SCIENCES, vol. 19, 1997, pages 744-752, XP009075826 * page 745, alinéa 2.1.1 * * page 746, alinéa 2.2 * ----- | 1-7 | INV. A61K8/97 A61K36/28 A61K36/41 A61Q19/00 |
| Y,D | MOMPON B ET AL: "CONFIGURATION OF SUBLUTEOLIDE A NEW GUAIANOLIDE ISOLATED FROM VERNONIA-SUBLUTEA COMPOSITAE" TETRAHEDRON, vol. 33, no. 17, 1977, pages 2199-2204, XP002410335 ISSN: 0040-4020 * le document en entier * ----- | 1-7 | |
| Y | WO 01/76551 A1 (LMD [FR]; JEAN DANIEL [FR]; CARIEL LEON [FR]) 18 octobre 2001 (2001-10-18) * page 1, alinéa 1 * * page 2, alinéa 2 - page 3, alinéa 2 * * page 4, alinéa 2 - alinéa 6; revendications * ----- | 1-7 | DOMAINES TECHNIQUES RECHERCHES (IPC): A61K |
| Y | US 5 057 501 A (THORNFELDT CARL R [US]) 15 octobre 1991 (1991-10-15) * colonne 2, ligne 62 - colonne 3, ligne 18; revendications * * colonne 5, ligne 49 - ligne 65 * ----- | 1-7 | |
| Y | EP 0 428 773 A (DERMATOLOGIC RESEARCH CORP [US]) 29 mai 1991 (1991-05-29) * page 2, ligne 50 - page 3, ligne 30; revendications; exemples * ----- | 1-7 | |

-/--

Le présent rapport a été établi pour toutes les revendications

| Lieu de la recherche | Date d'achèvement de la recherche | Examinateur |
|---|---|---|
| Munich | 23 juillet 2007 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 07 00 9359

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | TADEG H ET AL: "Antimicrobial activities of some selected traditional Ethiopian medicinal plants used in the treatment of skin disorders"<br>JOURNAL OF ETHNOPHARMACOLOGY, ELSEVIER SCIENTIFIC PUBLISHERS LTD, IE,<br>vol. 100, no. 1-2,<br>22 août 2005 (2005-08-22), pages 168-175, XP004996523<br>ISSN: 0378-8741<br>1. Introduction;<br>3. Results and discusion<br>----- | 2 | |
| Y | PURKAYASTHA J ET AL: "Ethnobotany of medicinal plants from Dibru-Saikhowa Biosphere Reserve of Northeast India"<br>FITOTERAPIA, IDB HOLDING, MILAN, IT,<br>vol. 76, no. 1, janvier 2005 (2005-01), pages 121-127, XP004722492<br>ISSN: 0367-326X<br>* page 126; tableau 1 *<br>----- | 3 | DOMAINES TECHNIQUES RECHERCHES (IPC) |
| A | DATABASE BIOSIS [Online]<br>BIOSCIENCES INFORMATION SERVICE, PHILADELPHIA, PA, US; août 2005 (2005-08),<br>MANJUNATHA B K ET AL: "Evaluation of wound-healing potency of Vernonia arborea Hk."<br>XP002410402<br>Database accession no. PREV200600136726<br>* abrégé *<br>& INDIAN JOURNAL OF PHARMACOLOGY,<br>vol. 37, no. 4, août 2005 (2005-08), pages 223-226,<br>ISSN: 0253-7613<br>----- | 1-7 | |

Le présent rapport a été établi pour toutes les revendications

| Lieu de la recherche | Date d'achèvement de la recherche | Examinateur |
|---|---|---|
| Munich | 23 juillet 2007 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

6

**ANNEXE AU RAPPORT DE RECHERCHE EUROPEENNE RELATIF A LA DEMANDE DE BREVET EUROPEEN NO.** EP 07 00 9359

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-07-2007

| Document brevet cité au rapport de recherche | | Date de publication | Membre(s) de la famille de brevet(s) | | Date de publication |
|---|---|---|---|---|---|
| WO 0176551 | A1 | 18-10-2001 | AU | 4663101 A | 23-10-2001 |
| | | | FR | 2807319 A1 | 12-10-2001 |
| US 5057501 | A | 15-10-1991 | AUCUN | | |
| EP 0428773 | A | 29-05-1991 | AU | 620937 B2 | 27-02-1992 |
| | | | AU | 4467589 A | 01-08-1991 |
| | | | US | 4978676 A | 18-12-1990 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **BABALOLA O.O.** *Afr. J. Med. Med. Sci.,* 2001, 91-93
- **MAZUMDER U.K.** *Phytomedicine,* 2003, 185-188
- **LEITE S.N.** *Fitoterapia,* 2002, vol. 73, 496-500
- **MOMPON B. ; TOUBIANA R.** *Tetrahedron,* 1976, vol. 33, 2199-2203
- **MOURÃO R.H.** *Phytotherapy Research,* 1999, 352-354